(19) Europäisches Patentamt
European Patent Office
Office européen des brevets

(11) Numéro de publication: **0 200 657 B1**

(12) # FASCICULE DE BREVET EUROPEEN

(45) Date de publication du fascicule du brevet: **17.11.88**

(51) Int. Cl.⁴: **C 07 C 43/225**, C 07 C 149/34, C 07 C 41/16

(21) Numéro de dépôt: **86420090.2**

(22) Date de dépôt: **27.03.86**

(54) **Procédé de préparation de trifluoroéthoxy ou trifluoroéthylthiobenzènes.**

(30) Priorité: **29.03.85 FR 8504758**

(43) Date de publication de la demande: **05.11.86 Bulletin 86/45**

(45) Mention de la délivrance du brevet: **17.11.88 Bulletin 88/46**

(84) Etats contractants désignés: **AT BE CH DE FR GB IT LI LU NL SE**

(56) Documents cités:
**FR - A - 2 529 543**
**GB - A - 2 045 760**

**SYNTHESIS, no. 9, septembre 1980, pages 727-728, Georg Thieme Verlag, Stuttgart, DE; F. CAMPS et al.: "A simple method for preparation of aryl 2,2,2-trifluoroethyl ethers"**
**BULLETIN OF THE CHEMICAL SOCIETY OF JAPAN, vol. 50, no. 11, novembre 1977, pages 3069-3070; T. NAKAI et al.: "A convenient preparation of arylthioynamines"**

(73) Titulaire: **RHONE-POULENC CHIMIE, 25, quai Paul Doumer, F-92408 Courbevoie Cédex (FR)**

(72) Inventeur: **Langlois, Bernard, 91, rue Duguesclin, F-69006 - Lyon (FR)**

(74) Mandataire: **Le Pennec, Magali et al, RHONE-POULENC INTERSERVICES Service Brevets Chimie 25, Quai Paul Doumer, F-92408 Courbevoie Cédex (FR)**

**EP 0 200 657 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne un procédé de préparation de trifluoroéthoxy ou trifluoroéthylthiobenzènes comme défini dans les revendications.

Il est connu d'après le brevet français n° 2 468 576 de préparer des dérivés trifluoroéthoxybenzéniques par réaction d'un phénol avec du trifluorométhanesulfonate de 2,2,2 trifluoroéthyle ou par réaction de dibromobenzène avec du trifluoroéthanol en présence d'hydrure de sodium et de catalyseur au cuivre.

Le premier procédé présente l'inconvénient d'utiliser comme matière première, le trifluorométhane sulfonate de 2,2,2 trifluoroéthyle qui n'est pas disponible en grande quantié sur le marché, ce qui grève au niveau industriel le prix d'obtention du trifluoroéthoxybenzène de manière irrémédiable.

Le second procédé a d'une part, l'inconvénient de présenter dans certains cas une mauvaise reproductibilité industrielle et exige d'autre part, l'utilisation de solvant aprotique onéreux dont le recyclage est difficile ce qui limite son utilisation industrielle.

Il est encore connu d'après Synthesis 1980 (9), 727-8 de préparer les 2,2,2 trifluoroéthylaryléthers par condensation d'un phénol avec du méthanesulfonate de 2,2,2 trifluoroéthyle en précense d'une base forte dans l'hexaméthylphosphorotriamide. Ce dernier solvant ne peut être utilisé dans l'industrie à cause de sa toxicité.

Il est connu d'après le «Bulletin de la société chimique du Japon» vol. 50, n° 11, pages 3069-3070 (1977) de préparer des trifluoroéthylthiobenzènes par mise en contact de thiophénol avec un trifluoroéthyltoluène sulfonate en présence d'amidure de sodium, la réaction ayant lieu dans un solvant aprotique dipolaire.

On préfère tout particulièrement utiliser les phénols ou thiophénols de formule (III) dans lesquels n = 1 ou 2.

On préfère tout particulièrement utiliser comme dérivé de formule générale (I), le paratoluènesulfonate de 2,2,2 trifluoroéthyle et le trichlorométhane sulfonate de 2,2,2 trifluoroéthyle.

Il est encore plus particulièrement avantageux d'utiliser le paratoluènesulfonate de 2,2,2 trifluoroéthyle.

La base mise en oeuvre dans le procédé de l'invention est choisie parmi le groupe comprenant les hydroxydes et les carbonates des métaux alcalins et alcalino-terreux. On peut citer notamment comme exemples de tels composés: la soude, la potasse, le carbonate de sodium ou de potassium et la chaux.

Selon un mode de réalisation préférentiel de l'invention, on utilise un agent séquestrant de formule (II) dans laquelle $R_1$, $R_2$, $R_3$ et $R_4$ représentant un atome d'hydrogène ou un radical méthyle, $R_5$ et n ayant la signification donnée dans la revendication 1.

Parmi ces derniers, on préfère encore plus particulièrement mettre en oeuvre les agents séquestrants pour lesquels n est supérieur ou égal à 0 et inférieur ou égal à 6 pour lesquels $R_5$ représente un radical alkyle ayant de 1 à 4 atomes de carbone.

On peut citer:

- la tris(oxa-3 butyl)amine de formule: $N\text{-}(CH_2\text{-}CH_2\text{-}O\text{-}CH_3)_3$
- la tris(dioxa-3,6 heptyl)amine de formule: $N\text{-}(CH_2\text{-}CH_2\text{-}O\text{-}CH_2\text{-}CH_2\text{-}O\text{-}CH_3)_3$
- la tris(trioxa-3,6,9 décyl)amine de formule: $N\text{-}(CH_2\text{-}CH_2\text{-}O\text{-}CH_2\text{-}CH_2\text{-}O\text{-}CH_2\text{-}CH_2\text{-}O\text{-}CH_3)_3$
- la tris(dioxa-3,6 octyl)amine de formule: $N\text{-}(CH_2\text{-}CH_2\text{-}O\text{-}CH_2\text{-}CH_2\text{-}O\text{-}C_2H_5)_3$
- la tris(trioxa-3,6,9 undécyl)amine de formule: $N\text{-}(CH_2\text{-}CH_2\text{-}O\text{-}CH_2\text{-}CH_2\text{-}O\text{-}CH_2\text{-}CH_2\text{-}O\text{-}C_2H_5)_3$
- la tris(dioxa-3,6 nonyl)amine de formule: $N\text{-}(CH_2\text{-}CH_2\text{-}O\text{-}CH_2\text{-}CH_2\text{-}O\text{-}C_3H_7)_3$
- la tris(trioxa-3,6,9 dodécyl)amine de formule: $N\text{-}(CH_2\text{-}CH_2\text{-}O\text{-}CH_2\text{-}CH_2\text{-}O\text{-}CH_2\text{-}CH_2\text{-}O\text{-}C_3H_7)_3$
- la tris(dioxa-3,6 décyl)amine de formule: $N\text{-}(CH_2\text{-}CH_2\text{-}O\text{-}CH_2\text{-}CH_2\text{-}O\text{-}C_4H_9)_3$
- la tris(trioxa-3,6,9 tridécyl)amine de formule: $N\text{-}(CH_2\text{-}CH_2\text{-}O\text{-}CH_2\text{-}CH_2\text{-}O\text{-}CH_2\text{-}CH_2\text{-}O\text{-}C_4H_9)_3$
- la tris(tétraoxa-3,6,9,12 tridécyl)amine de formule: $N\text{-}[CH_2\text{-}CH_2\text{-}O\text{-}(CH_2\text{-}CH_2\text{-}O)\text{-}_3\ CH_3]_3$
- la tris(hexaoxa-3,6,9,12,15,18 nonadécyl)amine de formule: $N\text{-}[CH_2\text{-}CH_2\text{-}O\text{-}(CH_2\text{-}CH_2\text{-}O)\text{-}_5\ CH_3]_3$
- la tris(dioxa-3,6 méthyl-4-heptyl)amine de formule: $N\text{-}[CH_2\text{-}CH_2\text{-}O\text{-}CH(CH_3)\text{-}CH_2\text{-}O\text{-}CH_3]_3$
- la tris(dioxa-3,6 diméthyl-2,4 heptyl)amine de formule: $N\text{-}[CH_2\text{-}CH(CH_3)\text{-}O\text{-}CH(CH_3)CH_2\text{-}O\text{-}CH_3]_3$

Les amines utilisées dans le procédé selon l'invention sont connues en tant que telles dans l'art antérieur. C'est ainsi que le brevet français n° 1 302 365 cite l'obtention des amines tertiaires $N(CH_2\text{-}CH_2\text{-}O\text{-}CH_3)_3$ et $N\text{-}(CH_2\text{-}CH_2\text{-}O\text{-}CH_2\text{-}CH_2\text{-}O\text{-}CH_3)_3$ comme sous produits de la synthèse des amines primaires et secondaires correspondantes.

Selon le mode particulier de mise en oeuvre de l'invention, on utilise un tiers solvant. Celui-ci doit répondre à certaines conditions: il doit solubiliser l'agent séquestrant et être inerte chimiquement vis-à-vis des composés à dissoudre. Ce solvant est choisi parmi les solvants aprotiques apolaires ou peu polaires comme par exemple: le chlorobenzène, l'orthodichlorobenzène, le trichloro-1,2,4 benzène, le tétrachlorure de carbone. Ces solvants sont tous peu onéreux.

Le trichlorobenzène est utilisé de préférence.

On peut ne pas utiliser de tiers solvant et c'est alors le phénol ou le thiophénol qui joue le rôle de solvant. On préfère utiliser un solvant quand le phénol ou le thiophénol a un point de fusion trop élevé ou quand il est trop réactif.

Le rapport molaire du phénol ou du thiophénol au dérivé de formule (I) est de préférence un rapport molaire compris entre 0,7 et 1,5.

Selon un mode particulier de mise en oeuvre de l'invention, la base est utilisée, en quantité telle que le rapport molaire de la base au phénol ou au thiophénol est compris entre 0,5 et 1,1.

Le rapport molaire de l'amine de formule générale (II) au phénol de formule générale (III) est de préférence compris entre 0,01 et 0,2.

La température à laquelle est soumise le mileu

réactionnel est de préférence comprise entre 80 et 200°C.

La réaction est de préférence mise en oeuvre à la pression atmosphérique mais une pression supérieure n'est nullement exclue du domaine de l'invention. La pression sera simplement adaptée à l'économie du procédé.

L'isolement des produits finaux est réalisé par filtration puis distillation sans aucun traitement accessoire à partir du mélange réactionnel ce qui est un avantage par rapport aux procédés de l'art antérieur.

Parmi les composés obtenus selon le procédé de l'invention, on peut citer: le (trifluoro 2,2,2 éthoxy)-benzène, les chloro, méthyle, méthoxy, nitro, cyano, trifluorométhyl, fluoro (trifluoro 2,2,2 éthoxy)- benzènes, le (trifluoro 2,2,2 éthoxy) 2' naphtalène, le (trifluoro- 2,2,2 éthylthio)benzène, les chloro, nitro (trifluoro-2,2,2 éthylthiobenzènes).

Les trifluoroéthoxy ou (trifluoroéthylthio)benzènes de la présente invention sont utilisés comme intermédiaires de synthèse pour la préparation de dérivés à activité pharmaceutique, vétérinaire ou phytosanitaire et dans l'industrie des lubrifiants (FR-A 2 468 576).

L'invention va être maintenant plus complètement décrite à l'aide des exemples qui vont suivre.

*Synthèse du chloro-4(trifluoro-2,2,2 éthoxy) benzène*

Dans un ballon de 500 ml équipé d'une agitation mécanique, d'un thermomètre et d'une petite colonne à distiller de type Vigreux, on charge:

- 200 ml de trichloro-1,2,4 benzène
- 4 g (0,1 mole) de soude finement broyée
- 1,6 g ($5 \cdot 10^{-3}$) de tris-(dioxa-3,6 heptyl)amine
- 12,9 g (0,1 mole) de parachlorophénol.

Cette suspension est agitée et portée à 100°C, température à partir de laquelle l'eau commence à distiller. Le milieu est maintenu entre 100 et 130°C jusqu'à cessation de la distillation de l'eau formée.

On refroidit alors vers 30°C et on introduit dans le mélange réactionnel 28,2 g (0,1 mole) de trichlorométhanesulfonate de trifluoro-2,2,2 éthyle.

On remplace la colonne à distiller par un réfrigérant ascendant et on porte, sous agitation, le milieu réactionnel à 145°C, température que l'on maintient pendant 4 heures.

L'analyse chromatographique en phase vapeur, avec étalon interne, indique alors un rendement brut en chloro-4(trifluoro-2,2,2 éthoxy)benzène de 58%.

Le mélange obtenu après refroidissement est filtré et le filtrat est soumis à la distillation sous pression réduite à l'aide d'une colonne à bande tournante.

On recueille ainsi 11,6 g de chloro-4(trifluoro-2,2,2 éthoxy)benzène représentant un rendement de 55% ($Eb_{25}$: 77-77,5°C).

*Autres exemples*

Ils sont résumés dans le tableau suivant:

TABLEAU I

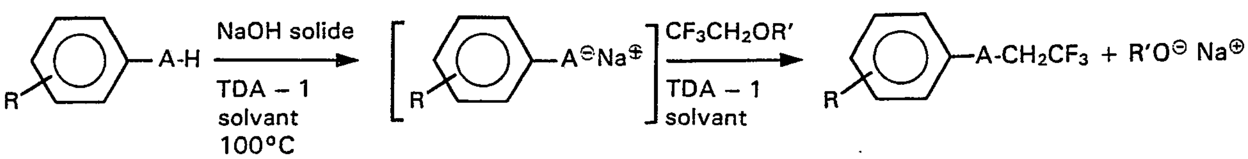


| R | A | Solvant | R' | Ar-A-H $CF_3CH_2OR'$ mole/mole | NaOH Ar-AH moles/ moles | TDA-1 Ar-A-H % moles | [Ar-A-H] moles/l | T°C | Durée (h) | Rdt Ar-A-$CH_2CF_3$ % |
|---|---|---|---|---|---|---|---|---|---|---|
| H | O | TCB | TsO | 1 | 1 | 5 | 0,4 | 170°C | 6 | 31% brut<br>21% distillé |
| $CH_3$-4 | O | TCB | $CCl_3SO_2$ | 0,7 | 1 | 4 | 0,5 | 145°C | 4 | 59% distillé |
| $CH_3O$-4 | O | TCB | $CH_3SO_2$ | 1 | 1 | 5 | 0,33 | 140°C | 6 | 21% brut |
| Cl-2 | O | TCB | $CCl_3SO_2$ | 1 | 1 | 5 | 0,5 | 145°C | 4 | 61% distillé |
| Cl-4 | O | TCB | $CCl_3SO_2$ | 1,2 | 0,85 | 5 | 0,5 | 145°C | 4 | 58% brut<br>55% distillé |
| Cl-4 | O | TCB | TsO | 1 | 1 | 5 | 0,5 | 150°C<br>+186°C<br>+186°C | 6<br>6<br>2 | 27% brut<br>57% brut<br>70% brut |
| H | S | TCB | $CF_3CO$ | 1 | 1 | 7,6 | 0,7 | 120-140 °C | 12 | 34% distillé |
| $C_{10}H_7OH$-2 (III) | | TCB | TsO | 1 | 1 | 15 | 0,5 | 140°C<br>+160°C | 12<br>+6 | } 20% cristallisé |

TCB = trichloro-1,2,4 benzène TsO = $CH_3$-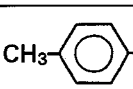-$SO_2$ TDA-1 = $N(CH_2\text{-}CH_2\text{-}O\text{-}CH_2\text{-}CH_2\text{-}O\text{-}CH_3)_3$

**Revendications**

1. Procédé de préparation de dérivés trifluoroéthoxyaromatiques ou de dérivés trifluoroéthylthioaromatiques caractérisé en ce qu'on fait reagir un dérivé aromatique hydroxylé ou comportant un groupe thiol de formule générale (III)

$R_n$-Ar-AH (III)

dans laquelle:

— Ar représente un noyau aromatique mono, polycyclique ou hétérocyclique,

— A représente le soufre ou l'oxygène,

— R représente au moins un substituant choisi parmi les radicaux suivants: hydrogène, halgoène, alkyle, alcoxy, alkylthio, phénoxy, nitro, alcoxycarbonyle, halogénoalkyle, halogénoalcoxy, halogénoalkylthio, phényle, benzoyle, cayano,

— n représente un nombre entier égal ou supérieur à 1 et égal ou inférieur à 5, avec un dérivé de formule générale (I):

$CF_3CH_2OR'$ (I)

dans laquelle R' représente un substituant choisi parmi le groupe comprenant le trifluoroacétyle, le méthanesulfonyle, le paratoluène sulfonyle, le trichlorométhanesulfonyle et le chlorosulfonyle en présence d'une base alcaline forte, solide choisie parmi le groupe comprenant les hydroxydes, et les carbonates de métaux alcalins ou alcalino-terreux et d'au moins un agent séquestrant de formule:

$N[CHR_1-CHR_2-O-(CHR_3-CHR_4-O)_n-R_5]_3$ (II)

dans laquelle n est un nombre entier supérieur ou égal à 0 et inférieur ou égal à 10 ($0 \leq n \leq 10$), $R_1$, $R_2$, $R_3$ et $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical $-C_mH_{2m}-C_6H_5$ ou $C_mH_{2m+1}-C_6H_4-$, où m est compris entre 1 et 12 en présence d'un solvant aprotique apolaire ou peu polaire.

2. Procédé selon la revendication 1, caractérisé en ce que dans la formule (III), n est égal à 1 ou 2.

3. Procédé selon la revendication 1, caractérisé en ce que dans la formule générale (I) R' représente un substituant choisi parmi le groupe comprenant le paratoluènesulfonyle et le trichlorométhanesulfonyle.

4. Procédé selon la revendication 1, caractérisé en ce que dans la formule (II), $R_1$, $R_2$, $R_3$ et $R_4$ représentent un atome d'hydrogène ou un radical méthyle.

5. Procédé selon la revendication 1, caractérisé en ce que dans la formule (II), n est un nombre entier supérieur ou égal à 0 et inférieur ou égal à 6.

6. Procédé selon la revendication 1, caractérisé en ce que dans la formule (II), $R_5$ représente un radical alkyle ayant de 1 à 4 atomes de carbone.

7. Procédé selon la revendication 1, caractérisé en ce que dans la formule (II), $R_1$, $R_2$, $R_3$ et $R_4$ identiques ou différents représentent un atome d'hydrogène ou un radical méthyle, n est un nombre entier supérieur ou égal à 0 et inférieur ou égal à 6 et $R_5$ représente un radical alkyle ayant de 1 à 4 atomes de carbone.

8. Procédé selon la revendication 7, caractérisé en ce que l'agent séquestrant de formule (II) est la tris(dioxa-3,6 heptyl)amine de formule: $N-(CH_2-CH_2-O-CH_2-CH_2-O-CH_3)_3$.

9. Procédé selon la revendication 1, caractérisé en ce que le rapport molaire du dérivé aromatique hydroxylé ou comportant un groupe thiol au dérivé de formule (I) est compris entre 0,5 et 5 et de préférence entre 0,7 et 1,5.

10. Procédé selon la revendication 1, caractérisé en ce que le rapport molaire de la base au dérivé aromatique hydroxylé est compris entre 0,5 et 1,1.

11. Procédé selon la revendication 1, caractérisé en ce que le rapport molaire du dérivé de formule (II) au dérivé aromatique hydroxylé est compris entre 0,01 et 0,2.

12. Procédé selon la revendication 1, caractérisé en ce que le solvant est choisi parmi le chlorobenzène, l'orthodichlorobenzène, le trichlorobenzène et le tétrachlorure de carbone.

13. Procédé selon la revendication 12, caractérisé en ce que le solvant choisi est le trichlorobenzène.

14. Procédé selon la revendication 1, caractérisé en ce que la température de la réaction est comprise entre 80 et 200°C.

**Patentansprüche**

1. Verfahren zur Herstellung von trifluorethoxyaromatischen oder trifluorethylthioaromatischen Derivaten, dadurch gekennzeichnet, dass man ein hydroxyliertes oder eine Thiolgruppe enthaltendes aromatisches Derivat der allgemeinen Formel (III)

$R_n$-Ar-AH (III)

in der

— Ar einen mono-, poly- oder heterocyclischen aromatischen Ring darstellt,

— A für Schwefel oder Sauerstoff steht,

— R für mindestens einen unter den folgenden Radikalen ausgewählten Substituenten: Wasserstoff, Halogen, Alkyl, Alkoxy, Alkylthio, Phenoxy, Nitro, Alkoxycarbonyl, Halogenalkyl, Halogenalkoxy, Halgenalkylthio, Phenyl, Benzoyl, Cyan,

— n eine ganze Zahl gleich oder grösser als 1 und gleich oder kleiner als 5 ist,

mit einem Derivat der allgemeinen Forme (I):

$CF_3CH_2OR'$ (I)

in der R' einen Substituenten aus der Trifluoracetyl, Methansulfonyl, Paratoluolsulfonyl, Trichloromethansulfonyl und Chlorsulfonyl umfassenden Gruppe darstellt,

in Anwesenheit einer stark alkalischen, festen Base aus der Gruppe der Hydroxyde und Karbonate der Alkali- oder Erdalkalimetalle und mindestens eines Komplexbildners der Formel

$N[CHR_1-CHR_2-O-(CHR_3-CHR_4-O)_n-R_5]_3$ (II)

in der n eine ganze Zahl grösser oder gleich 0 und kleiner oder gleich 10 ($0 \leq n \leq 10$), $R_1$, $R_2$, $R_3$ und $R_4$ gleich oder verschieden sind und ein Wasserstoffatom oder einen Alkylrest mit 1 bis 4 Kohlenstoffatomen darstellen und

$R_5$ einen Alkyl- oder Cycloalkylrest mit 1 bis 12 Kohlenstoffatomen, einen Phenylrest oder einen der Reste $-C_mH_{2m}-C_6H_5$ und $C_mH_{2m+1}-C_6H_4-$ bedeutet, in denen m zwischen 1 und 12 liegt,

in einem unpolaren oder wenig polaren, aprotischen Lösungsmittel umsetzt.

2. Verfahren nach Anspruch 1, dadurch gekennzeichnet, dass in der Formel (III) n gleich 1 oder 2 ist.

3. Verfahren nach Anspruch 1, dadurch gekennzeichnet, dass in der allgemeinen Formel (I) R' einen Substituenten aus der Paratoluolsulfonyl und Trichlormethansulfonyl umfassenden Gruppe darstellt.

4. Verfahren nach Anspruch 1, dadurch gekennzeichnet, dass in der Formel (II) $R_1$, $R_2$, $R_3$ und $R_4$ ein Wasserstoffatom oder einen Methylrest bedeuten.

5. Verfahren nach Anspruch 1, dadurch gekennzeichnet, dass in der Formel (II) n eine ganze Zahl grösser oder gleich 0 und kleiner oder gleich 6 ist.

6. Verfahren nach Anspruch 1, dadurch gekennzeichnet, dass in der Formel (II) $R_5$ einen Alkylrest mit 1 bis 4 Kohlenstoffatomen bedeutet.

7. Verfahren nach Anspruch 1, dadurch gekennzeichnet, dass in der Formel (II) $R_1$, $R_2$, $R_3$ und $R_4$ gleich oder verschieden sind und ein Wasserstoffatom oder einem Methylrest bedeuten, n eine ganze Zahl grösser oder gleich 0 und kleiner oder gleich 6 ist und $R_5$ einen Alkylrest mit 1 bis 4 Kohlenstoffatomen darstellen.

8. Verfahren nach Anspruch 7, dadurch gekennzeichnet, dass der Komplexbildner der Formel (II) Tris(3,6-dioxa-heptyl)amin der Formel $N-(CH_2-CH_2-O-CH_2-CH_2-O-CH_3)_3$ ist.

9. Verfahren nach Anspruch 1, dadurch gekennzeichnet, dass das Molverhältnis von dem hydroxylierten oder eine Thio-Gruppe tragenden aromatischen Derivat zu dem Derivat der Formel (I) zwischen 0,5 und 5 und vorzugsweise zwischen 0,7 und 1,5 liegt.

10. Verfahren nach Anspruch 1, dadurch gekennzeichnet, dass das Molverhältnis der Base zu dem hydroxylierten, aromatischen Derivat zwischen 0,5 und 1,1 liegt.

11. Verfahren nach Anspruch 1, dadurch gekennzeichnet, dass das Molverhältnis des Derivats der Formel (II) zum hydroxylierten, aromatischen Derivat zwischen 0,01 und 0,2 liegt.

12. Verfahren nach Anspruch 1, dadurch gekennzeichnet, dass das Lösungsmittel unter Chlorbenzol, Orthodichlorbenzol, Trichlorbenzol und Tetrachlorkohlenstoff ausgewählt wird.

13. Verfahren nach Anspruch 12, dadurch gekennzeichnet, dass das gewählte Lösungsmittel Trichlorbenzol ist.

14. Verfahren nach Anspruch 1, dadurch gekennzeichnet, dass die Reaktionstemperatur zwischen 80 und 200°C liegt.

## Claims

1. Process for the preparation of trifluoroethoxyaromatic derivatives or trifluoroethylthioaromatic derivatives, characterized in that an aromatic derivative which is either hydroxylated or which comprises a thiol group of general formula (III)

$R_n-Ar-AH$ (III)

in which formula:

— Ar denotes a monocyclic, polycyclic or heterocyclic aromatic nucleus,

— A denotes sulphur or oxygen,

— R denotes at least one substituent chosen from the following radicals: hydrogen, halogen, alkyl, alkoxy, alkylthio, phenoxy, nitro, alkoxycarbonyl, haloalkyl, haloalkoxy, haloalkylthio, phenyl, benzoyl and cyano, and

— n denotes an integer equal to or greater than 1 and equal to or less than 5,

is reacted with a derivative of general formula (I):

$CF_3CH_2OR'$ (I)

in which formula R' denotes a substituent chosen from the group comprising trifluoroacetyl, methanesulphonyl, paratoluenesulphonyl, trichloromethanesulphonyl and chlorosulphonyl, in the presence of a solid, strongly alkaline base chosen from the group comprising alkali metal or alkaline-earth metal hydroxides and carbonates and at least one sequestering agent of formula:

$N[CHR_1-CHR_2-O-(CHR_3-CHR_4-O)_n-R_5]_3$ (II)

in which formula n is an integer greater than or equal to 0 and less than or equal to 10 ($0 \leq n \leq 10$), $R_1$, $R_2$, $R_3$ and $R_4$, which are identical or different, denote a hydrogen atom or an alkyl radical containing from 1 to 4 carbon atoms and $R_5$ denotes an alkyl or cycloalkyl radical containing from 1 to 12 carbon atoms, a phenyl radical or a $-C_mH_{2m}-C_6H_5$ or $C_mH_{2m+1}-C_6H_4-$ radical, where m is from 1 to 12

in the presence of an apolar or weakly polar aprotic solvent.

2. Process according to Claim 1, characterized in that, in formula (III), n is equal to 1 or 2.

3. Process according to Claim 1, characterized in that, in general formula (I), R' denotes a substituent chosen from the group comprising para-toluenesulphonyl and trichloromethanesulphonyl.

4. Process according to Claim 1, characterized in that, in formula (II), $R_1$, $R_2$, $R_3$ and $R_4$ denote a hydrogen atom or a methyl radical.

5. Process according to Claim 1, characterized in that, in formula (II), n is an integer greater than or equal to 0 and less than or equal to 6.

6. Process according to Claim 1, characterized in that, in formula (II), $R_5$ denotes an alkyl radical containing from 1 to 4 carbon atoms.

7. Process according to Claim 1, characterized in that, in formula (II), $R_1$, $R_2$, $R_3$ and $R_4$, which are identical or different, denote a hydrogen atom or a methyl radical, n is an integer greater than or equal

to 0 and less than or equal to 6 and $R_5$ denotes an alkyl radical containing from 1 to 4 carbon atoms.

8. Process according to Claim 7, characterized in that the sequestering agent of formula (II) is tris-(3,6-dioxa-heptyl)amine of formula:
$N-(CH_2-CH_2-O-CH_2-CH_2-O-CH_3)_3$.

9. Process according to Claim 1, characterized in that the molar ratio of the aromatic derivative which is either hydroxylated or which comprises a thiol group to the derivative of formula (I) is between 0.5 and 5 and preferably between 0.7 and 1.5.

10. Process according to Claim 1, characterized in that the molar ratio of the base to the hydroxylated aromatic derivative is between 0.5 to 1.1.

11. Process according to Claim 1, characterized in that the molar ratio of the derivative of formula (II) to the hydroxylated aromatic derivative is between 0.01 to 0.2.

12. Process according to Claim 1, characterized in that the solvent is chosen from chlorobenzene, ortho-dichlorobenzene, trichlorobenzene and carbon tetrachloride.

13. Process according to Claim 12, characterized in that the solvent chosen is trichlorobenzene.

14. Process according to Claim 1, characterized in that the reaction temperature is between 80 and 200°C.